(19) Europäisches Patentamt
European Patent Office
Office européen des brevets

(11) Veröffentlichungsnummer : **0 010 671**
**B1**

(12) **EUROPÄISCHE PATENTSCHRIFT**

(45) Veröffentlichungstag der Patentschrift : **11.11.81**

(51) Int. Cl.³ : **C 07 D487/08**// B01J21/06 ,(C07D487/08, 241/00, 241/00)

(21) Anmeldenummer : **79103963.9**

(22) Anmeldetag : **15.10.79**

# (54) Verfahren zur Herstellung von 1,4-Diazabicyclo-(2,2,2)-octan.

(30) Priorität : **27.10.78 DE 2846813**

(43) Veröffentlichungstag der Anmeldung : **14.05.80 (Patentblatt 80/10)**

(45) Bekanntmachung des Hinweises auf die Patenterteilung : **11.11.81 Patentblatt 81/45**

(84) Benannte Vertragsstaaten : **BE CH DE FR GB IT NL**

(56) Entgegenhaltungen : **DE - A - 1 445 578**

(73) Patentinhaber : **BAYER AG**
**Zentralbereich Patente, Marken und Lizenzen**
**D-5090 Leverkusen 1, Bayerwerk (DE)**

(72) Erfinder : **Imre, Laszlo, Dr.**
**Franz-Marc-Strasse 16**
**D-5090 Leverkusen (DE)**
Erfinder : **Horstmann, Walter, Dr.**
**Eichenweg 17**
**D-5060 Bergisch Gladbach 2 (DE)**
Erfinder : **Leopold, Hans-Gerhard**
**Pionierstrasse 4**
**D-5000 Köln 60 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung von 1,4-Diazabicyclo-(2,2,2)-octan

Die Erfindung betrifft ein Verfahren zur Herstellung von 1,4-Diazabicyclo-(2,2,2)-octan aus Piperazinverbindungen.

Zur Herstellung von 1,4-Diazabicyclo-(2,2,2)-octan aus N-(β-Aminoethyl)-piperazin werden nach der US 3 157 657 γ-Aluminiumoxid, nach der US 3 056 788 10 %iges Wolframoxid auf Aluminiumoxid, nach der US 2 985 658 Aluminiumsilikat und nach Japan Kokai 7 558 096 synthetische Zeolithe als Katalysatoren verwendet. Als Katalysatoren werden ferner bei der Herstellung von 1,4-Diazabicyclo-(2,2,2)-octan aus N-(β-Hydroxyethyl)-piperazin oder aus N,N'-Bis-(β-hydroxyethyl)-piperazin nach der US 3 279 701 Metallphosphate, nach der US 3 285 920 Metalle an oxidischen Trägermaterialien, nach der US 3 166 558 Aluminiumsilikate, nach Japan Kokai 7 511 797 Aluminiumoxid oder Aluminiumsilikat und nach der DE-A 1 445 418 Aluminium-, Molybdän- und Magnesiumsilikate und Zeolithe verwendet. Weiterhin ist aus der DE-A 1 445 578 ein Verfahren zur Herstellung von 1,4-Diazabicyclo-(2,2,2)-octan aus N-(β-Hydroxyethyl)-piperazin oder N,N'-Bis-(β-hydroxyethyl)-piperazin bekannt, bei dem man die Ausgangsmaterialien gasförmig bei 300-600 °C über einen Katalysator aus säurebehandelten Bentoniten leitet.

Diese Verfahren sind sehr aufwendig, nachdem die Umsetzungen an den beschriebenen Katalysatoren sehr unspezifisch unter Bildung einer Vielzahl von Nebenprodukten, deren Abtrennung mit erheblichem Aufwand verbunden ist, verlaufen.

Solche Nebenprodukte sind beispielsweise Piperazin oder Ethanolamin, die man nach dem beschriebenen Verfahren in hoher Konzentration erhält. Das Verhältnis Piperazin zu 1,4-Diazabicyclo-(2,2,2)-octan betrug 1: 3 bis 1: 1 (vgl. Japan Kokai 7558096, USP 3 279 701). Aus solchen Gemischen läßt sich 1,4-Diazabicyclo-(2,2,2)-octan nicht wirtschaftlich isolieren.

1,4-Diazabicyclo-(2,2,2)-octan wird mit ungenügender Reinheit erhalten.

Es wurde ein Verfahren zur Herstellung von 1,4-Diazabicyclo-(2,2,2)-octan durch katalytische Umsetzung von Piperazinverbindungen bei erhöhter Temperatur in der Gasphase gefunden, das dadurch gekennzeichnet ist, daß man Piperazinverbindungen der Formel

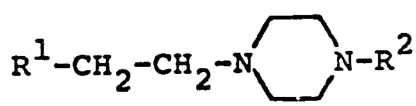

worin

$R^1$ die Hydroxyl- oder die Aminogruppe bedeutet und

$R^2$ für Wasserstoff oder den Rest $—CH_2—CH_2—R^3$ steht,

wobei $R^3$ eine Hydroxyl- oder eine Aminogruppe sein kann, im Gemisch mit einem Inertgas in Gegenwart eines Siliciumdioxid enthaltenden Katalysators, der einen Gehalt an freiem $SiO_2$ nach dem Glühen bei 800 °C von mindestens 97 Gew.-% hat und dessen innere Oberfläche nach BET größer als 30 $m^2/g$ ist, bei Temperaturen von 200 bis 500 °C und einem Druck von 0,01-100 bar behandelt.

Das erfindungsgemäße Verfahren kann am Beispiel der folgenden Reaktionsgleichung erläutert werden :

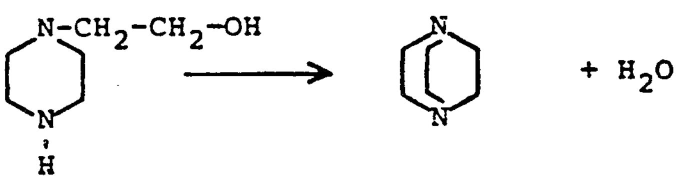

Als Piperazinverbindungen seien im einzelnen genannt : N-(β-Aminoethyl)-piperazin, N,N'-Bis-(β-aminoethyl)-piperazin, N-(β-hydroxyethyl)-piperazin und N,N'-Bis-(β-hydroxyethyl)-piperazin sowie N-(β-Hydroxyethyl)-N'-(β-aminoethyl)-piperazin, wobei die 4 erstgenannten Verbindungen bevorzugt sind. N-(β-hydroxyethyl)-piperazin ist als Ausgangsmaterial besonders bevorzugt.

Die Herstellung der Piperazinverbindungen für das erfindungsgemäße Verfahren ist an sich bekannt (Ber. dtsch. Ges. *59,* 2 423 (1926) ; Am. Soc. *68,* 1 296 (1946) ; US-3 089 876 ; US-3 031 452).

Bei dem erfindungsgemäßen Verfahren werden $SiO_2$ enthaltende Katalysatoren eingesetzt, deren Gehalt an freiem $SiO_2$ nach dem Glühen bei 800 °C mindestens 97 Gew.-% beträgt und deren innere Oberfläche größer als 30 $m^2/g$ nach BET (vgl. Brunnauer, Emmet und Teller, Journal of the American Chemical Society, *60* (1938), 309) ist. Bevorzugt sind Katalysatoren mit einer inneren Oberfläche von 50 bis 800 $m^2/g$, besonders bevorzugt von 80 bis 500 $m^2/g$, jeweils nach BET. Die Katalysatoren können bis zu 3 Gew.-% andere Oxide, wie beispielsweise MgO, CaO, $Fe_2O_3$, $K_2O$ und $Na_2O$ und außerdem Spuren von NaCl und/oder $NaSO_4$, enthalten. Beispielsweise sind die nach dem in der Deutschen Offenlegungsschrift 1 767 754 beschriebenen Verfahren hergestellten Kieselsäureprodukte als Katalysatoren für das erfindungsgemäße Verfahren geeignet. Entsprechend dieser Offenlegungsschrift werden poröse, abriebfeste, perlförmige, vorwiegend Kieselsäure enthaltende Produkte erhalten, indem man Feststoffe in

einem wäßrigen stabilen Kieselsäuresol mit einer spezifischen Oberfläche von 150 bis 400 $m^2/g$ nach BET suspendiert, die erhaltene Suspension mit einer wäßrigen Anschlämmung von hydratisiertem Magnesiumoxid in Mengen von 0,1-3 Gew.-% (bezogen auf das wasserfreie Granulat) vermischt, diese gelierfähige Mischung zu Tropfen der gewünschten Größe verteilt, die Tropfen in einer mit Wasser nicht mischbaren Flüssigkeit geliert, das Granulat von der Flüssigkeit abtrennt, trocknet und glüht, wobei in dem Kieselsäuresol ein Kieselsäurehaltiger Füllstoff mit einer spezifischen Oberfläche von 20 bis 200 $m^2/g$ nach BET in Mengen von 20 bis 60 Gew.-% (bezogen auf das trockene Granulat) und Tonmineralien aus der Gruppe Kaolinit, Montmorillonit und Attapulgit in Mengen von 5 bis 30 Gew.-% suspendiert werden, die erhaltene Suspension durch Zusatz des hydratisierten, feinteiligen Magnesiumoxids und Verteilung der Suspension in ein mit Wasser nicht mischbares Medium zu einem perlförmigen Granulat geliert und das Granulat anschließend trocknet und mindestens 10 Minuten bei Temperaturen von 500 bis 1 000 °C härtet. Bei einer thermischen Dauerbelastung bis etwa 750 °C verändern sich die chemischen und physikalischen Eigenschaften dieser Kieselsäure-Produkte nicht (Erdöl und Kohle, *23,* 648 bis 651 (1970)).

Weitere mögliche Herstellungsverfahren des Siliciumdioxid enthaltenden Katalysators sind in Ullmanns Encyklopädie der technischen Chemie 3. Auflage, Band 15, Seiten 712-732 (1964) zusammengestellt. Man geht meistens von Kieselsäuren aus (Kieselsol Kieselgel, Kieselgur), die durch Eintropfen, Einrühren oder Einsprühen in mit Wasser nicht mischbare Flüssigkeiten, z.B. in Paraffinöl, Körner bilden. Sie werden bei 100 °C getrocknet und nach Glühen bei 800-1 000 °C wasserfrei erhalten.

Der Katalysator kann in verschiedener Form vorliegen, beispielsweise als Pulver, in grobkörniger Form oder in Form von Kugeln, Würstchen, Pillen oder Zylindern, und in einem Festbett, oder zur besseren Wärmeabführung, in einem Wirbelbett oder einem anderen bewegten Katalysatorbett. Beim Arbeiten in Wirbelbett sind beispielsweise kugelförmige Katalysatorteilchen mit einem Durchmesser von 0,01 bis 2 mm bevorzugt. Im Festbett können beliebig geformte Katalysatorteilchen mit einer Teilchengröße von im allgemeinen etwa 0,2 bis 20 mm verwendet werden. Bevorzugt sind auch Würstchen mit einer Länge von 4 bis 18 mm oder Kugeln mit einem Durchmesser von 0,4 bis 10 mm.

Für das erfindungsgemäße Verfahren wird besonders bevorzugt reines Siliciumdioxid als Katalysator verwendet, das wie folgt charakterisiert ist :

Innere Oberfläche 80 bis 500 $m^2/g$ nach BET, kugelförmige Teilchen mit einem Durchmesser von 0,4 bis 4 mm und Anordnung des Katalysators in einem Festbett oder in einem Wirbelbett.

Sollte der Katalysator durch Abscheidung von Verunreinigungen in seiner Aktivität nachlassen, kann man durch Abbrennen in Gegenwart von Luft bei erhöhter Temperatur, beispielsweise bei 400 bis 750 °C, die Ausgangsaktivität wieder herstellen.

Das erfindungsgemäße Verfahren wird in der Gasphase durchgeführt. Innerhalb des Bereiches für das Arbeiten in der Gasphase können Temperatur, Zusammensetzung des Gasgemisches und Strömungsgeschwindigkeit in weiten Grenzen variiert werden.

Das erfindungsgemäße Verfahren wird bei 0,01 bis 100 bar, also bei Normaldruck, vermindertem Druck oder erhöhtem Druck durchgeführt. Vorzugsweise wird bei Drucken von 0,03 bis 30 bar gearbeitet.

Die Reaktionstemperaturen für das erfindungsgemäße Verfahren liegen im allgemeinen im Temperaturbereich von 200 bis 500 °C. Bevorzugt wird im Temperaturbereich von 240 bis 350 °C gearbeitet.

Die Piperazinverbindungen werden im Gemisch mit Inertgasen eingesetzt. Inertgase für das erfindungsgemäße Verfahren sind Edelgase, Stickstoff, Wasserdampf und/oder Wasserstoff und/oder Ammoniak. Bevorzugte Inertgase sind Stickstoff und/oder Wasserdampf und/oder Ammoniak.

Bevorzugt werden für das erfindungsgemäße Verfahren Piperazinverbindungen im Gemisch mit den Inertgasen in Konzentration von 2 bis 50 Vol.-%, besonders bevorzugt von 5 bis 25 Vol.-%, eingesetzt.

Die Strömungsgeschwindigkeit des Gasgemisches im Reaktor kann in weiten Grenzen variiert werden. Für das erfindungsgemäße Verfahren wird bevorzugt die Strömungsgeschwindigkeit so reguliert, daß sich Kontaktzeiten im Reaktionsraum von 0,01 bis 50 sek, besonders bevorzugt von 1 bis 10 sek, berechnet für die Katalysatorschüttung bei gegebener Reaktionstemperatur, ergeben.

Entsprechend den unterschiedlichen Möglichkeiten zur Anordnung des Katalysators kann der Reaktor verschieden ausgebildet sein. Wird mit einem Festbett-Katalysator gearbeitet, so kann der Katalysator beispielsweise in einem oder mehreren Reaktionsrohren fest angeordnet sein (Röhrenreaktor). Die Reaktionsrohre können aus Metall, einer keramischen Masse oder aus Glas oder Quarz bestehen. Bevorzugt werden Metallrohre, beispielsweise aus Stahl verwendet. Wird der Katalysator in Form eines Wirbelbetts angewandt, so wird zweckmäßigerweise ein Wirbelbettreaktor üblicher Bauart (Verfahrenstechnik katalytischer Reaktionen in Katalysatoren, Tenside und Mineralöladditive, G. Thieme Verlag Stuttgart (1978)) verwendet. Die Zuführung der Einsatzprodukte zum Reaktor und ihre Aufheizung kann auf verschiedene Weise vorgenommen werden. Beispielsweise kann man die Piperazinverbindungen mit Stickstoff direkt im Verdampfer mischen und das so erhaltene Einsatzgemisch in einem Wärmeaustauscher zunächst auf beispielsweise 240 bis 300 °C vorerhitzen und dann dem Reaktor zuleiten, wo es gegebenenfalls weiter bis auf die Reaktionstemperatur erhitzt wird.

Man kann aber das Aufheizen der Reaktanten auch getrennt vornehmen und diese getrennt dem Reaktor zuführen.

Besonders bevorzugt für das erfindungsgemäße Verfahren ist es, wenn die Umsetzung so geführt wird, daß nur eine partielle Umsetzung stattfindet. Bevorzugt sind in diesem Falle Umsetzungen von 2 bis

80 %, bevorzugt von 5 bis 50 %. Bei dieser Verfahrensweise werden besonders hohe Selektivitäten erreicht.

Die den Reaktor verlassenden Gase bestehen im allgemeinen aus 1,4-Diazabicyclo-(2,2,2)-octan, nicht umgesetzten Piperazinverbindungen und den Inertgasen. Als Nebenprodukte können höhermolekulare Verbindungen anfallen, die von dem erfindungsgemäß hergestellten 1,4-Diazabicyclo-(2,2,2)-octan sehr leicht abgetrennt werden können. Auf diese Weise erhält man nach dem erfindungsgemäßen Verfahren 1,4-Diazabicyclo-(2,2,2)-octan in hoher Reinheit.

Die Abtrennung des 1,4-Diazabicyclo-(2,2,2)-octans aus der Reaktionsmischung kann nach bekannten Verfahren (z.B. fraktionierte Destillation, Sublimation, Umkristallisation) erfolgen. Man kann beispielsweise die vom Katalysator abgeschiedenen Dämpfe durch Kühlung kondensieren und das Kondensat bei Normaldruck fraktioniert destillieren. Die Kühlung der Dämpfe kann beispielsweise im Temperaturbereich von −50 bis +50 °C, zweckmäßigerweise etwa bei 0 °C erfolgen. Durch fraktionierte Destillation erhält man dann im Siedebereich von 160 bis 180 °C und bei einem Druck von 1 bar eine im wesentlichen 1,4-Diazabicyclo-(2,2,2)-octan enthaltende Fraktion, die durch erneute Destillation und/oder Sublimation in ein hochreines Produkt überführt werden kann.

Die Reinigung des 1,4-Diazabicyclo-(2,2,2)-octans kann auch entsprechend der US 2 937 176 erfolgen, indem man das Produkt mit einem flüssigen, niedrig siedenden, aliphatischen Kohlenwasserstoff, wie n-Pentan, verreibt und kühlt.

Es ist auch möglich, das noch verunreinigte 1,4-Diazabicyclo-(2,2,2)-octan aus Ethylacetat umzukristallisieren.

In einer bevorzugten Ausführungsform des erfindungsgemäßen Verfahrens wird wie folgt gearbeitet : Die Piperazinverbindung wird in einem Verdampfer bei etwa 240 °C verdampft und mit Wasserdampf vermischt, so daß man ein Gasgemisch mit der Zusammensetzung von 5 bis 25 Vol.-% der Piperazinverbindung und 95 bis 75 Vol.-% an Wasserdampf erhält. Das so erhaltene Gasgemisch wird gegebenenfalls in einem Vorerhitzer auf die Reaktionstemperatur von 240 bis 350 °C gebracht und bei etwa 1 bar über den aus Siliciumdioxid bestehenden Katalysator geleitet. Die Strömungsgeschwindigkeit wird so eingestellt, daß man Kontaktzeiten zwischen 1 und 10 Sekunden erhält. Das den Reaktor verlassende Gasgemisch wird auf 0 °C abgekühlt und das Kondensat destillativ und durch Umkristallisation aufgearbeitet. Das nach der Abtrennung des gebildeten 1,4-Diazabicyclo-(2,2,2)-octan verbleibende Restgas und das nicht umgesetzte Ausgangsprodukt wird wieder in die Reaktion zurückgeführt. Der verbrauchte Anteil an Ausgangsprodukt wird dabei zweckmäßigerweise ersetzt.

Das erfindungsgemäße Verfahren kann diskontinuierlich und kontinuierlich durchgeführt werden.

Nach dem erfindungsgemäßen Verfahren kann 1,4-Diazabicyclo-(2,2,2)-octan in einfacher Weise mit hoher Selektivität und hohen Ausbeuten hergestellt werden.

Die Umsetzung nach dem erfindungsgemäßen Verfahren ist überraschend, weil sich 1,4-Diazabicyclo-(2,2,2)-octan in einfacher Weise an wohlfeilen Katalysatoren mit sehr guter Reinheit herstellen läßt und vielfach zur sofortigen technischen Weiterverarbeitung geeignet ist.

1,4-Diazabicyclo-(2,2,2)-octan kann bei der Herstellung von Polyurethanen verwendet werden (Ullmanns Enzyklopädie der Technischen Chemie, Band 7, Seite 386, 4. Auflage, (1974)).

Für die folgenden Beispiele werden die Begriffe Umsatz und Selektivität wie folgt definiert :

$$\% \text{ Umsatz} = \frac{\text{Mol umgesetzte Ausgangsverbindung}}{\text{Mol eingesetzte Ausgangsverbindung}} \cdot 100$$

$$\% \text{ Selektivit} = \frac{\text{Mol gebildetes 1,4-Diazabicyclo-(2,2,2)-octan}}{\text{Mol umgesetzte Ausgangsverbindung}} \cdot 100$$

## Beispiel 1

In ein elektrisch beheiztes, senkrecht angeordnetes Reaktionsrohr von 50 cm Länge und 2,40 cm Durchmesser, das aus Glas besteht, werden 14,0 $cm^3$ Siliciumdioxid als Katalysator eingefüllt. Der Katalysator besteht aus reinen $SiO_2$-Körnern. Die spezifische Oberfläche beträgt 110 $m^2/g$. Die Körner besitzen eine Kugelgestalt mit abriebfester Oberfläche. Die Korngrößenverteilung liegt im Bereich von 0,4 bis 2 mm Durchmesser. Die Länge der Katalysatorstrecke beträgt 3 cm. Diese Strecke hat eine homogene Temperatur, die auf 275 °C gehalten wird. In dieser Temperaturzone läuft die Reaktion ab.

Es werden in ein Verdampfungsgefäß mit einem Volumen von etwa 100 $cm^3$ bei 240 °C stündlich 7 g N-(β-Hydroxyethyl)-piperazin eindosiert und mit 5 N l Stickstoff bei 1 bar Gesamtdruck vermischt. Es entspricht einer Ausgangszusammensetzung von 19,7 Vol.-% N-(β-Hydroxyethyl)-piperazin und 80,3 % Stickstoff. Das so erhaltene Gasgemisch wird über den Katalysator geleitet. Aus den angegebenen Zahlenwerten errechnet sich eine Durchsatzgeschwindigkeit von 3,46 $cm^3$/sek und eine Kontaktzeit von 4,1 sek. Die den Reaktor verlassenden Dämpfe des Gasstromes werden bei 0 °C durch Kühlung kondensiert.

Zur Gewinnung des Diazabicyclooctans aus der Reaktionsmischung wird zunächst die im Verlaufe von 20 Stunden erhaltene, kondensierte Phase bei 1 bar fraktioniert destilliert. Im Siedebereich von 160 bis 180 °C wird eine an Diazabicyclooctan reiche Fraktion als Rohprodukt (17,8 g) erhalten.

Die gaschromatografische Untersuchung dieser Fraktion nach der Methode des inneren Standards an 10 % CARBOWAX 20 M auf CHROMOSORB W zeigte neben dem nicht umgesetzten N-(β-Hydroxyethyl)-piperazin praktisch nur 1,4-Diazabicyclo-(2,2,2)-octan (99,6 %) als Produkt. Dies entspricht einer Selektivität von 56,3 % bei einem Umsatz von 26,2 %. Das nicht umgesetzte N-(β-Hydroxyethyl)-piperazin kann für weitere Umsetzung zurückgeführt werden. Die Roh-Fraktion kann aus Ethylacetat umkristallisiert werden, wobei man 14,6 g Diazabicyclooctan in reiner Form erhält.

## Beispiele 2 bis 10

Es wurde wie in Beispiel 1 gearbeitet, jedoch wurden andere Kieselgelprodukte mit mindestens 99,5 % $SiO_2$-Gehalt in geglühtem Zustand als Katalysatoren verwendet. Die jeweilige Gaszusammensetzung, Kontaktzeit, Temperatur, Katalysatoroberfläche und die bei 1 bar erhaltenen Umsätze und Selektivitäten sind aus der folgenden Tabelle 1 ersichtlich, wobei die Selektivitäten aus den tatsächlich durch Umkristallisation aus Ethylacetat erhaltenen Diazabicyclooctanmengen errechnet wurden.

Tabelle 1

| Beispiel Nr. | Kieselgel-oberfläche ($m^2/g$) | Temperatur (°C) | Kontaktzeit (sec) | Gaszusammensetzung HAEP | $N_2$ | $H_2O$ (Vol. %) | Umsatz (%) | Selektivität (%) |
|---|---|---|---|---|---|---|---|---|
| 2 | 480 | 285 | 1,7 | 19,7 | 80,3 | — | 33,8 | 54,7 |
| 3 | 620 | 315 | 0,8 | 12,1 | — | 87,9 | 25,3 | 58,0 |
| 4 | 180 | 320 | 4,3 | 24,0 | — | 76,0 | 22,8 | 54,0 |
| 5 | 440 | 260 | 10,2 | 19,7 | 80,3 | — | 30,8 | 47,6 |
| 6 | 110 | 290 | 2,1 | 19,7 | 80,3 | — | 21,8 | 63,9 |
| 7 | 110 | 280 | 2,3 | 10,9 | 89,1 | — | 22,6 | 69,3 |
| 8 | 270 | 275 | 1,2 | 5,3 | — | 94,7 | 14,4 | 71,2 |
| 9 | 90 | 350 | 0,5 | 9,4 | 45,3 | 45,3 | 27,4 | 48,1 |
| 10 | 270 | 240 | 12,0 | 27,8 | 71,2 | — | 16,4 | 42,5 |

HAEP = N-(β-Hydroxyäthyl)-piperazin

## Beispiel 11

Es wird in ein Druckrohr von 32 cm Länge und 1,8 cm Durchmesser aus V4A-Stahl eine Kieselgelfüllung von 5 cm Länge eingebracht. Der $SiO_2$-Gehalt liegt über 99,5 %. Die spezifische Oberfläche beträgt etwa 500 $cm^2/g$ die Körnung 1,5 bis 3 mm. Über diesen Katalysator wird bei 8 bar und einer Temperatur von 250 °C und 11 l/h eines Gasgemisches von folgender Zusammensetzung geleitet :

19,7 Vol.-% N-(β-Hydroxyäthyl)-piperazin, 80,3 Vol.-% $N_2$. Unter diesen Bedingungen erreicht man eine Selektivität von 59,2 % bei einem Umsatz von 27,8 %.

## Beispiel 12

Es wird wie in Beispiel 11 gearbeitet, jedoch wird in das Verdampfungsgefäß stündlich 7 g N,N'-Bis-(β-hydroxyäthyl)-piperazin gelöst in 15 g Wasser eindosiert. Das erhaltene Gasgemisch wird dann bei 65 mbar und 310 °C über den Katalysator geleitet. Das den Reaktor verlassende Gasgemisch wird durch Kühlung kondensiert und gaschromatographisch analysiert. Es wird eine Selektivität von 44,7 % erreicht, d.h. daß 44,7 % des umgesetzten N,N'-Bis-(β-hydroxyäthyl)-piperazin zu Diazabicyclooctan umgewandelt worden sind. Der Umsatz beträgt 32,3 %.

## Beispiel 13

Es wird in der gleichen Apparatur wie in Beispiel 1, beschrieben gearbeitet, jedoch wird ein Gasstrom aus 19,7 Vol.-% N-(β-Aminoäthyl)-piperazin und 80,3 Vol.-% Sticksoff erzeugt und über den Katalysator geleitet. Unter den gleichen Bedingungen wie in Beispiel 1 wird ein Umsatz von 23,0 % und eine Selektivität von 32,7 % erzielt.

Vergleichsbeispiel 1 (gemäß DE-A 1 445 418)

Es wurde wie im Beispiel 1 gearbeitet, jedoch wurde ein handelsübliches Zeolith 4 Å als Katalysator verwendet. Dabei wurde 1,4-Diazabicyclo-(2,2,2)-octan mit einer Selektivität von 28,7 % bei einem Umsatz von 33,6 % erhalten. Außerdem wurde viel Piperazin gefunden. Das Verhältnis Piperazin zu 1,4-Diazabicyclo-(2,2,2)-octan betrug 1: 2,7.

Vergleichsbeispiel 2 (gemäß Japan Kokai 75 11 797)

Es wurde wie in Biespiel 1 gearbeitet, jedoch wurde ein handelsübliches Aluminiumsilikat mit der Zusammensetzung 87,3 % $SiO_2$, 12,4 % $Al_2O_3$, 0,25 % $Na_2O$ und einer BET-Oberfläche von 290 $m^2/g$ als Katalysator verwendet. Die erzielte Selektivität betrug 34,2 % bei einem Umsatz von 38,5 %. Das Verhältnis Piperazin zu 1,4-Diazabicyclo-(2,2,2)-octan betrug ferner 1: 2,3.

Vergleichsbeispiel 3 (gemäß DE-A 1 445 578)

Die Nacharbeitung von Beispiel 1 ergab nach Destillation 62 g nicht destillierbaren Rückstand. Durch fraktionierte Destillation wurden 3 Fraktionen erhalten :

1. Fraktion : Kp. 80 bis 160 °C 148 g
2. Fraktion : Kp. 160 bis 180 °C 224 g
3. Fraktion : Kp. 180 bis 250 °C 84 g

Nach gaschromatographischer Analyse (Methode des inneren Standards analog erfindungsgemäßen Beispiel 1) enthält die Fraktion 2 84 % Diazabicyclooctan sowie beträchtliche Mengen an Nebenprodukte, wie Piperazin, von denen das Diazabicyclooctan nur mit einem hohen technischen Aufwand abgetrennt werden kann.

Der Gesamtumsatz bezogen auf eingesetztes Produkt betrug 80,6 % der Theorie.

**Ansprüche**

1. Verfahren zur Herstellung von 1,4-Diazabicyclo-(2,2,2)-octan durch katalytische Behandlung von Piperazinverbindungen bei erhöhter Temperatur in der Gasphase, dadurch gekennzeichnet, daß man Piperazinverbindungen der Formel

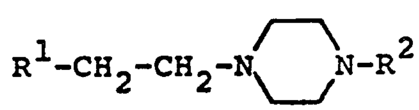

worin

$R^1$ die Hydroxyl- oder die Aminogruppe bedeutet und

$R^2$ für Wasserstoff oder den Rest $—CH_2—CH_2—R^3$ steht, wobei

$R^3$ eine Hydroxyl- oder eine Aminogruppe sein kann, im Gemisch mit einem Inertgas in Gegenwart eines Siliciumdioxid enthaltenden Katalysators, der einen Gehalt an freiem $SiO_2$ nach dem Glühen bei 800 °C von mindestens 97 Gew.-% hat und dessen innere Oberfläche nach BET größer als 30 $m^2/g$ ist, bei Temperaturen von 200 bis 500 °C und einem Druck von 0,01 bis 100 bar behandelt.

2. Verfahren nach Anspruch 1, dadurch gekennzeichnet, daß man als Katalysator reines Siliciumdioxid verwendet.

3. Verfahren nach den Ansprüchen 1 und 2, dadurch gekennzeichnet, daß man die Behandlung im Druckbereich von 0,03 bis 30 bar durchführt.

4. Verfahren nach den Ansprüchen 1 bis 3, dadurch gekennzeichnet, daß die Kontaktzeit der Piperazinverbindungen am Katalysator 0,01 bis 50 sekt beträgt.

5. Verfahren nach den Ansprüchen 1 bis 4, dadurch gekennzeichnet, daß als Inertgas Wasserdampf und/oder Stickstoff und/oder Ammoniak verwendet wird.

6. Verfahren nach den Ansprüchen 1 bis 5, dadurch gekennzeichnet, daß man N-(β-Hydroxyethyl)-piperazin als Piperazinverbindung einsetzt.

**Claims**

1. Process for the preparation of 1,4-diazabicyclo-(2,2,2)-octane by catalytic treatment of piperazine compounds at elevated temperature in the gas phase, characterised in that piperazine compounds of the formula

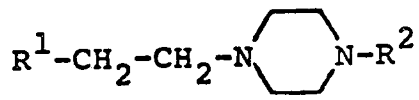

wherein

$R^1$ denotes the hydroxyl group or the amino group and

$R^2$ represents hydrogen or the radical $-CH_2-CH_2-R^3$ wherein

$R^3$ may be a hydroxyl group or an amino group, in a mixture with an inert gas, are treated in the presence of a catalyst containing silicon dioxide, which catalyst has a content of free $SiO_2$, after calcining at 800 °C, of at least 97 % by weight and a BET internal surface area of more than 30 $m^2/g$, at temperatures of 200 to 500 °C and under a pressure of 0.01 to 100 bars.

2. Process according to Claim 1, characterised in that pure silicon dioxide is used as the catalyst.

3. Process according to Claims 1 and 2, characterised in that the treatment is carried out in the pressure range from 0.03 to 30 bars.

4. Process according to Claims 1 to 3, characterised in that the contact time of the piperazine compounds on the catalyst is 0.01 to 50 seconds.

5. Process according to Claims 1 to 4, characterised in that steam and/or nitrogen and/or ammonia is used as the inert gas.

6. Process according to Claims 1 to 5, characterised in that N-(β-hydroxyethyl)-piperazine is employed as the piperazine compound.

**Revendications**

1. Procédé de préparation de 1,4-diazabicyclo-(2,2,2)-octane par traitement catalytique de composés de pipérazine à température élevée et en phase gazeuse, caractérisé en ce qu'on traite des composés de pipérazine de formule :

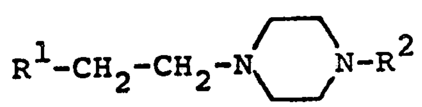

dans laquelle

$R^1$ représente le groupe hydroxy ou le groupe amino, et

$R^2$ représente un atome d'hydrogène ou le radical $-CH_2-CH_2-R^3$,

$R^3$ pouvant être un groupe hydroxy ou un groupe amino, en mélange avec un gaz inerte et en présence d'un catalyseur contenant du dioxyde de silicium et ayant une teneur en $SiO_2$ libre d'au moins 97 % en poids après calcination à 800 °C, la surface interne de ce catalyseur (suivant Brunnauer, Emmet et Teller) étant supérieure à 30 $m^2/g$, ce traitement étant effectué à des températures de 200 à 500 °C et sous une pression de 0,01 à 100 bars.

2. Procédé suivant la revendication 1, caractérisé en ce que, comme catalyseur, on utilise le dioxyde de silicium pur.

3. Procédé suivant les revendications 1 et 2, caractérisé en ce qu'on effectue le traitement sous une pression se situant dans l'intervalle allant de 0,03 à 30 bars.

4. Procédé suivant les revendications 1 à 3, caractérisé en ce que le temps de contact des composés de pipérazine sur le catalyseur est de 0,01 à 50 secondes.

5. Procédé suivant les revendications 1 à 4, caractérisé en ce que, comme gaz inertes, on utilise la vapeur d'eau et/ou l'azote et/ou l'ammoniac.

6. Procédé suivant les revendications 1 à 5, caractérisé en ce que, comme composé de pipérazine, on utilise la N-(β-hydroxyéthyl)-pipérazine.